Europäisches Patentamt

European Patent Office

Office européen des brevets

(11) Publication number: **0 209 128 B1**

(12) **EUROPEAN PATENT SPECIFICATION**

(45) Date of publication of patent specification: **02.09.92**  (51) Int. Cl.⁵: **A61K 31/34**

(21) Application number: **86109775.6**

(22) Date of filing: **16.07.86**

(54) **Oxygenated fluorocarbon for use in intra peritoneal perfusions.**

(30) Priority: **19.07.85 US 757015**

(43) Date of publication of application:
**21.01.87 Bulletin 87/04**

(45) Publication of the grant of the patent:
**02.09.92 Bulletin 92/36**

(84) Designated Contracting States:
**AT BE CH DE FR GB IT LI LU NL SE**

(56) References cited:
**US-A- 4 445 886**

**B.R. ANAESTH., vol. 56, 1984, pages 867-872
N.S. FAITHFULL et al.: "Whole body oxygen-
ation using intraperitoneal perfusion of
fluorocarbons"**

(73) Proprietor: **THOMAS JEFFERSON UNIVERSITY
11th and Walnut Streets
Philadelphia Pennsylvania 19107(US)**

(72) Inventor: **Osterholm, Jewell L.
579 Huston Road
Radnor Pennsylvania(US)**

(74) Representative: **Baillie, Iain Cameron et al
c/o Ladas & Parry, Altheimer Eck 2
W-8000 München 2(DE)**

Rank Xerox (UK) Business Services

## Description

This invention relates generally to artificial respiratory devices and to compositions for use in methods, more particularly in chemical methods for providing whole body oxygenation of a mammal whose respiratory system is partially or completely inoperative. More particularly, the present invention relates to compositions for use in methods and devices for treating mammals suffering from anoxia.

This application is related to U.S. Patent No. 4,445,500, U.S. Patent 4,445,886 and U.S. Patent 4,378,797.

The present application is also related to the following issued United States patents, U.S. Patent 4,445,514; U.S. Patent 4,393,863; U.S. Patent No. 4,450,841; U.S. Patent No. 4,445,887; U.S. Patent No. 4,446,154; U.S. Patent 4,446,155; U.S. Patent 4,451,251; U.S. Patent 4,445,888 and U.S. Patent 4,445,500.

There are many post-traumatic and post-operative patients who develop major pulmonary complications which interfere with or preclude adequate oxygenation. The "shock lung" best characterizes this syndrome complex. Severe pneumonias, smoke inhalation, acute respiratory obstructions, pre-mature birth, and birth-related pulmonary injury also can lead to the same general problems with oxygenation. Patients with massive pulmonary embolism and hemothorax also suffer from severe hypoxemia. Combining patients in these categories, there is a substantial population of patients at high risk, but whose conditions are potentially reversible, given adequate oxygenation.

The present invention utilizes an oxygenated fluorocarbon liquid for general body oxygenation, which is applied as a circulation through the peritoneal cavity. The aforementioned patents and patent applications reference in detail various prior art publications relating to fluorocarbons and their medical uses. More recently, in the British Journal of Anaesthesia 56: 867 (1984) in an article entitled "Whole Body Oxygenation Using Intra Peritoneal Perfusion of Fluorocarbons" by Faithfull, Klein, van der Zee and Salt, results of a preliminary study undertaken to assess the feasibility of increasing the arterial oxygen tension, and decreasing the arterial carbon dioxide tension, in intact animals, by means of peritoneal perfusion with the perfluorocarbon-containing, oxygen-transporting blood substitute, 20% Fluosol-DA, were disclosed. This British Journal of Anaesthesia article is not believed to be prior art to the present application.

See also U.S. Patent No. 4,402,984 (Moore).

The present invention provides the use of an oxygenated perfluorocarbon for preparing a medicament useful in a novel method of whole body oxygenation of the tissue of a living mammal comprising the steps of: providing an oxygenated fluorocarbon-containing liquid; injecting said oxygenated fluorocarbon liquid into the peritoneal cavity of said mammal; and withdrawing said fluorocarbon liquid from said cavity, said injecting and withdrawing being conducted at a rate sufficient to oxygenate at least a portion of the tissue of said mammal. According to the invention the oxygenated perfluorocarbon has a partial pressure of $CO_2$ in excess of 2.6 kPa (20 Torr). In accordance with the preferred embodiment of the present invention, an oxygenated fluorocarbon emulsion having an aqueous component, a fluorocarbon component, and an emulsification component is utilized which is oxygenated to a $pO_2$ in excess of 66 kPa (500 $mmH_g$) prior to injection. The preferred rate of injection is above 20 milliliters per minute per kilogram of body weight of said mammal, preferably about 25 milliliters per minute per kilogram of said body weight. In the preferred embodiment, the perfusion rate is selected to increase the arterial blood gas of said mammal. Accordingly, the method in which the present invention is involved provides a novel "artificial lung" which may be used to provide sufficient oxygen to the blood to maintain life even in the presence of complete or near complete respiratory failure.

These and other objects of the invention will become apparent from the following more detailed description.

The present invention is useful in a novel method for oxygenating the tissue of a living mammal employing an oxygenated fluorocarbon containing liquid. The preferred fluorocarbon containing liquid is the oxygenated fluorocarbon nutrient emulsion which is disclosed in my aforementioned United States patents, such as U.S. Patent 4,445,886, Although this fluorocarbon containing emulsion is presently preferred, it is anticipated that certain constituents presently contained in this emulsion may be eliminated from the fluorocarbon containing liquid used in accordance with the preferred embodiment of the present invention. For example, to minimize the likelihood of bacterial growth, glucose may be eliminated from the fluorocarbon emulsion formulation. Similarly, the subject amino acids and steroids in the subject composition may be eliminated, if desired. Although not presently preferred, it is within the scope of the said method to inject a liquid consisting essentially of the oxygenated fluorocarbon itself. In this treatment modality, it will be preferred to follow this fluorocarbon treatment with a lavage intended to wash remaining fluorocarbon from the peritoneum at the conclusion of treatment. This lavage may comprise injecting isotonic saline with or without a detergent or emulsifier, such as the pluronic disclosed in the aforementioned patent, to thereby reduce the likelihood of long term toxicity. Under most circumstances, it will be preferred that either or both of the subject perfusate and the subsequent lavage contain an antibiotic, such as bactracin, to minimize the incidence of peritoneal infection

The preferred fluorocarbon emulsion, as described in U.S. Patent 4,445,886, comprises a perfluorobutyl tetrahydrofuran, an emulsifier such as Pluronic electrolytes (Pluronic is a trademark), and various components such as glucose, amino acids, ions, hormones, vitamins, etc. The emulsion is oxygenatable to $po_2$ values of about 86, kPa (650 mmHg) using a pump oxygenator and about 53 kPa (400 mmHg) with simply bubble oxygenation. Frequently, the emulsion will also be treated with carbon dioxide to give $pCO_2$ values in the range 2.6-4 kPa (20-30 mmHg). The milliosmolarity of the subject emulsion is typically physiologic, normally having an mOsM/L of about 298-317, or about 310.

In accordance with the present invention, it is desired to provide carbon dioxide and oxygen to the subject emulsion in a pump-type oxygenator such as a Harvey Pediatric Oxygenator, to obtain $po_2$ values in excess of 66, kPa (500 mmHg), preferably 73-86 kPa (550-650 mmHg).

In order to ensure that substantial oxygen transfer will occur, it is presently preferred to oxygenate the subject fluorocarbon containing liquid to a $pO_2$ in excess of 66 kPa (500 mmHg) prior to injection. As reported in the aforementioned patents, these oxygen tensions are easily obtainable with the subject oxygenated fluorocarbon emulsion.

The preferred fluorocarbon emulsion, as described in U.S. Patent 4,445,886, comprises a perfluorobutyl tetrahydrofuran, an emulsifier such as a pluronic, electrolytes, and various components such as glucose, amino acids, ions, hormones, vitamins, etc. The emulsion is oxygenatable to $pO_2$ values of about 86 kPa (650 mmHg) using a pump oxygenator and about 52.8 kPa (400 mmHg) with simply bubble oxygenation. Frequently the emulsion will also be treated with carbon dioxide to give $pCO_2$ values in the range 2.6-4 kPa (20-30 mmHg). The milliosmolarity of the subject emulsion is typically physiologic, normally having an mOsM/L of about 298-317, or about 310.

In accordance wtih the present invention, it is desired to provide carbon dioxide and oxygen to the subject emulsion in a pump-type oxygenator such as a Harvey Pediatric Oxygenator, to obtain $pO_2$ values in excess of 66 kPa (500 mmHg), preferably 73-86 kPa (550-650 mmHg).

The present invention is illustrated by the following examples:

Experiments were begun using a male 5 kg (11 pound) orange tabby cat which was anesthetized using a 35 milligram per kilogram intra-muscular injection of ketamine. Thirty minutes later, 20 milligrams of flexedil, a respiratory paralytic was administered at 20 milligrams intravenously, and the animal then placed on a respirator. After 20 minutes, its arterial blood was determined to have a pH of 7.430, a $pCO_2$ 3.39 kPa of (25.5 mmHg) and a $pO_2$ of 13.7 kPa (103 mmHg). The aforementioned oxygenated nutrient emulsion (using the fluorocarbon FC-80) was placed in a Harvey pediatric oxygenator (volume 1230ml) and maintained at about 40°C. Large 1.3 to 1.9 cm (1/2 to 3/4 inch) cannulas were placed through two flank incisions into the peritoneum. A Randoff pump was initially used to inject the oxygenated fluorocarbon nutrient emulsion into the peritoneum through one of the cannulas, the second cannula being routed back to the oxygenator for recirculation and reoxygenation of the subject fluorocarbon emulsion. After 10 minutes of administration of a 90% $N_2O$ - 10% $O_2$ respiratory gas mixture, the pH of the arterial blood was determined to be 7.374, the $pCO_2$ to be 3.5 kPa (28.2 mmHg), and the $pO_2$ to be 6.4 kPa (48 mmHg). Perfusion of the peritoneal space was established at a flow rate greater than 200 milliliters per minute. Problems were encountered, however, with the patency of the return line. Apparently, fatty tissue was being drawn into the return line, a condition which persisted until the catheters were manipulated into the space below omentum, which resolved the problem. Before the collection of meaningful data could be obtained, however, an inadvertent disconnection of the respirator resulted in the expiration of the test animal. Accordingly, a second series of tests were performed using a male, white and grey, 4.3 kg (9 1/2 pound) cat. At 2:05 p.m. 150 milligrams of ketamine and 0.18 milligrams of atropine were administered intramuscularly. At 2:15 p.m. a 70/30 mixture of $N_2O/O_2$ was begun through a respirator. At 2:30 p.m. 20 milligrams of flexedil was administered. The arterial blood gas at 2:35 p.m. registered a $pCO_2$ of 5.75 kPa 43.2 mmHg, a $pO_2$ 41.6 kPa (313 mmHg); the pH was 7.283. The relatively higher small $pO_2$ of this arterial blood gas is considered within the normal range given the possibility of some hyperventilation and the administration of a respiratory gas containing 30% oxygen. At 2:35 p.m. the respirator was adjusted to increase the volume to 45 from 35. At 2:50 p.m. the arterial blood gas was 4.8 kPa (36 Torr) $pCO_2$, 40.7 kPa (306 Torr) $pO_2$, at a pH of 7.318. At 2:55 p.m. a 90/10 $N_2O/O_2$ mixture was substituted as the respiration gas. At 3:05 p.m. the aforementioned fluorocarbon emulsion from the Harvey pediatric oxygenator was determined to have an oxygen tension of 75.1 kPa (565 Torr), a carbon dioxide tension of 3.3 kPa (25 Torr), and a pH of 7.951. At 3:10 p.m. the arterial blood gas of the subject animal had an oxygen tension of 6.3 kPa (48 Torr), a carbon dioxide tension of 4.4 kPa (33.4 mmHg) and a pH of 7.335. At 3:15 p.m. the arterial blood gas of that animal exhibited a $pO_2$ tension of 3.7 kPa (28 mmHg), a carbon dioxide tension of 5.1 kPa (38.4 mmHg) and a pH of 7.354. At 3:24 p.m. the carbon dioxide tension was 5.1 kPa (43.5 mmHg), the oxygen tension 4.8 kPa (36 mmHg) and the pH 7.311. At 3:36 p.m. the carbon dioxide tension was 5.03 kPa (37.8 mmHg), the oxygen tension 4.8 kPa (36 mmHg), and the pH 7.292. At 3:58 p.m. the pH was 7.260, the carbon dioxide tension was 5.6 kPa (42.4 mmHg), and the oxygen tension was 4.5 kPa (34 mmHg). At 4:05 p.m. the oxygen tension of the injected and withdrawn fluorocarbons was determined. The fluorocarbon injected was determined to have an oxygen tension of 79 kPa (594 mmHg) and a pH of 6.815; the fluorocarbon withdrawn from the peritoneum was found to have a $pO_2$ of 68 kPa (511 mmHg) and a pH of 6.865. The carbon dioxide tension in the withdrawn fluid was determined to be 3.5 kPa (26.7 Torr), but was not determined for the input fluorocarbon at this time. At 4:15 p.m. the arterial blood gas was determined to have a pH of 7.212, a carbon dioxide tension of 5.4 kPa (40.7 Torr) and a $pO_2$ of 5.6 kPa (42 Torr). At 4:20 p.m. the fluorocarbon was determined to have pH of 7.612. Unfortunately, the $pO_2$ electrode used to determine oxygen tensions in this test was apparently poisoned by the fluorocarbon, and therefore provided doubtful accuracy. It is believed that it was recalibrated, and at 4:20 p.m. the arterial blood gas pH was found to be 7.170, the $pO_2$ tension to be 61 kPa (46 Torr), and the carbon dioxide tension to be 4.83 kPa (36.3 Torr). Return flow, i.e., withdrawn of the fluorocarbon containing liquid from the peritoneum, was improved in this test by routing the exit cannula to a ballast receptacle at atmospheric pressure which was then used as an intermediate reservoir to supply the oxygenator input. As seen from the above, a systemic arterial $pO_2$ of approximately 4 kPa (30 mmHg) (i.e., 3.7-4.8 kPa (28-36 mmHg)) was achieved by drastic hypoventilation. When oxygenated fluorocarbon was perfused through the cat peritoneum at rates of about 200 to 250 milliliters per minutes, this severe hypoxia was alleviated, as indicated by increased systemic $pO_2$ of about 6.1 kPa (46 mmHg).

Accordingly, the present invention has been demonstrated as being useful in treating systemic anoxia under conditions where the subject mammals respiratory system is not capable of providing normal arterial $pO_2$ tensions.

## Claims

1. Use of an oxygenated perfluorocarbon having a partial pressure of carbon dioxide in excess of 2.6 kPa (20 Torr) for preparing a medicament for treating a mammal in need of whole body oxygenation by injecting said oxygenated fluorocarbon liquid into the peritoneal cavity of said mammal and withdrawing said liquid from said cavity;

said injecting and withdrawing being conducted at a rate sufficient to oxygenate at least a portion of the tissue of said mammal said rate being at least 20 ml/kg body weight/minute.

2. A use as claimed in claim 1, wherein said oxygenated fluorocarbon liquid comprises an oxygenated fluorocarbon-containing aqueous emulsion.

3. A use as claimed in either of the preceding claims wherein said oxygenated perfluorocarbon has a partial pressure of oxygen in excess of 66 kPa (500 torr), preferably in the range 73 - 86 kPa (550-650 Torr).

4. A use as claimed in any one of the preceding claims wherein said oxygenated perfluorocarbon has a partial pressure of carbon dioxide in the range 2.6 to 4.0 kPa (20 to 30 Torr).

5. A use as claimed in any one of the preceding claims wherein said oxygenated perfluorocarbon has a milliosmolarity in the range 290-320.

6. A use as claimed in any one of the preceding claims wherein said oxygenated perfluorocarbon is perfluorobutyl tetrahydrofuran.

7. An oxygenated fluorocarbon liquid specifically adapted for oxygenating the tissue of a living mammal by injection into the peritoneal cavity of said mammal and withdrawing said liquid from said cavity, the injection and withdrawal being conducted at such a rate as to oxygenate at least a portion of the tissue of said mammal said oxygenated perfluorocarbon having a partial pressure of oxygen in excess of 66 kPa (500 Torr), preferably in the range 73-86 kPa (550-650 Torr) and a partial pressure of carbon dioxide in excess of 2.6 kPa (20 Torr).

8. An oxygenated fluorocarbon liquid according to claim 7, in the form of an aqueous emulsion.

9. An oxygenated fluorocarbon liquid as claimed in either of claims 7 and 8 which has a partial pressure of carbon dioxide in the range 2.6 to 4.0 kPa (20 to 30 Torr).

**10.** An oxygenated fluorocarbon liquid according to any of claims 7-9 which has a milliosmolarity in the range 290-320.

**11.** An oxygenated fluorocarbon liquid as claimed in any of claims 7-11 which is perfluorobutyl tetrahydrofuran.

**Patentansprüche**

**1.** Verwendung eines oxygenierten Perfluorkohlenstoffs mit einem Partialdruck des Kohlendioxids von über 20 Torr (2,6 kPa) zur Herstellung eines Medikaments für die Behandlung eines Säugetieres durch Injizieren des oxygenierten flüssigen Fluorkohlenstoffs in die Bauchfellhöhle dieses Säugetieres und Abziehen der Flüssigkeit aus dieser Höhle;
wobei das Injizieren und Abziehen mit einer Geschwindigkeit durchgeführt wird, die ausreicht, um wenigstens einen Teil des Gewebes des Säugetieres zu oxygenieren, wobei die Geschwindigkeit mindestens 20 ml/kg Körpergewicht/Minute beträgt.

**2.** Verwendung nach Anspruch 1, dadurch gekennzeichnet, daß der oxygenierte flüssige Fluorkohlenstoff eine oxygenierte, Fluorkohlenstoff enthaltende wäßrige Emulsion umfaßt.

**3.** Verwendung nach einem der vorhergehenden Ansprüche, dadurch gekennzeichnet, daß der oxygenierte Perfluorkohlenstoff einen Partialdruck des Sauerstoffs von über 500 Torr (66 kPa) besitzt, vorzugsweise im Bereich von 550-650 Torr (73 - 86 kPa).

**4.** Verwendung nach einem der vorhergehenden Ansprüche, dadurch gekennzeichnet, daß der oxygenierte Perfluorkohlenstoff einen Partialdruck des Kohlendioxids im Bereich von 20 bis 30 Torr (2.6 - 4 kPa) besitzt.

**5.** Verwendung nach einem der vorhergehenden Ansprüche, dadurch gekennzeichnet, daß der oxygenierte Perfluorkohlenstoff eine Milliosmolarität im Bereich von 290-320 besitzt.

**6.** Verwendung nach einem der vorhergehenden Ansprüche, dadurch gekennzeichnet, daß der oxygenierte Perfluorkohlenstoff Perfluorbutyltetrahydrofuran ist.

**7.** Oxygenierter flüssiger Perfluorkohlenstoff, der besonders geeignet ist, um das Gewebe eines lebenden Säugetieres zu oxygenieren, indem diese Flüssigkeit in die Bauchfellhöhle des Säugetieres injiziert und aus dieser Höhle abgezogen wird, wobei das Injizieren und Abziehen mit einer Geschwindigkeit durchgeführt wird, die ausreicht, um wenigstens einen Teil des Gewebes des Säugetieres zu oxygenieren, wobei der oxygenierte Perfluorkohlenstoff einen Partialdruck des Sauerstoffs von über 500 Torr (66 kPa) besitzt, vorzugsweise im Bereich von 550 - 650 Torr (73-86 kPa), und einen Partialdruck des Kohlendioxids von über 20 Torr (2,6 kPa).

**8.** Oxygenierter flüssiger Perfluorkohlenstoff nach Anspruch 7, in Form einer wäßrigen Emulsion.

**9.** Oxygenierter flüssiger Fluorkohlenstoff nach einem der Ansprüche 7 und 8, der einen Partialdruck des Kohlendioxids im Bereich von 20 bis 30 Torr (2,6 - 4 kPa) besitzt.

**10.** Oxygenierter flüssiger Fluorkohlenstoff nach einem der Ansprüche 7 bis 9, der eine Milliosmolarität im Bereich von 290 - 320 besitzt.

**11.** Oxygenierter flüssiger Fluorkohlenstoff nach einem der Ansprüche 7 bis 11, der Perfluorbutyltetrahydrofuran ist.

**Revendications**

**1.** Emploi d'un perfluorocarbone oxygéné ayant une pression partielle de dioxyde de carbone supérieure à 2,6 kPa (20 Torr) pour préparer un médicament destiné à traiter un mammifère nécessitant une oxygénation corporelle totale par injection dudit liquide de fluorocarbone oxygéné dans la cavité péritonéale dudit mammifère et retrait dudit liquide de ladite cavité;
ladite injection et ledit retrait étant opérés à un débit suffisant pour oxygéner au moins une partie des tissus dudit mammifère, ledit débit étant d'au moins 20 ml/kg de poids corporel par minute.

**2.** Emploi selon la revendication 1, dans lequel ledit liquide de fluorocarbone oxygéné comprend une émulsion aqueuse contenant du fluorocarbone oxygéné.

**3.** Emploi selon l'une ou l'autre des revendications précédentes, dans lequel ledit perfluorocarbone oxygéné a une pression partielle d'oxygène supérieure à 66 kPa (500 Torr), comprise de préférence dans la plage de 73 à 86 kPa (550 à 650 Torr).

**4.** Emploi selon l'une quelconque des revendications précédentes, dans lequel ledit perfluorocarbone oxygéné a une pression partielle de dioxyde de carbone comprise dans la plage de 2,6 à 4,0 kPa (20 à 30 Torr).

5. Emploi selon l'une quelconque des revendications précédentes, dans lequel ledit perfluorocarbone oxygéné a une milliosmolarité comprise dans la plage de 290 à 320.

6. Emploi selon l'une quelconque des revendications précédentes, dans lequel ledit perfluorocarbone oxygéné est du perfluorobutyltétrahydrofuranne.

7. Liquide de fluorocarbone oxygéné spécifiquement conçu pour oxygéner les tissus d'un mammifère vivant par injection dans la cavité péritonéale dudit mammifère et retrait dudit liquide de ladite cavité, l'injection et le retrait étant opérés à un débit propre à oxygéner au moins une partie des tissus dudit mammifère, ledit perfluorocarbone oxygéné ayant une pression partielle d'oxygène supérieure à 66 kPa (500 Torr), comprise de préférence dans la plage de 73 à 86 kPa (550 à 650 Torr) et une pression partielle de dioxyde de carbone supérieure à 2,6 kPa (20 Torr).

8. Liquide de fluorocarbone oxygéné selon la revendication 7, ayant la forme d'une émulsion aqueuse.

9. Liquide de fluorocarbone oxygéné selon l'une ou l'autre des revendications 7 et 8, ayant une pression partielle de dioxyde de carbone comprise dans la plage de 2,6 à 4,0 kPa (20 à 30 Torr).

10. Liquide de fluorocarbone oxygéné selon l'une quelconque des revendications 7 à 9, ayant une milliosmolarité comprise dans la plage de 290 à 320.

11. Liquide de fluorocarbone oxygéné selon l'une quelconque des revendications 7 à 11, qui est du perfluorobutyltétrahydrofuranne.